# EUROPEAN PATENT APPLICATION

(11) **EP 3 028 715 A1**
(43) Date of publication of application: **08.06.2016**
(21) Application number: 14829320.2
(22) Date of filing: 28.07.2014
(51) Int. Cl.: A61K 39/39, A61P 37/04

(54) **USE OF RAPAMYCIN AS VACCINE ADJUVANT AND PREPARATION METHOD THEREFOR**

(30) Priority: 26.07.2013 CN 201310320670
(71) Applicant: Institut Pasteur of Shanghai, Chinese Academy of Sciences, Shanghai 200031 (CN)
(72) Inventor: XIAO, Hui, Shanghai 200031 (CN); YUAN, Chuanping, Shanghai 200031 (CN); ZANG, Aiping, Shanghai 200031 (CN); DU, Min, Shanghai 200031 (CN)
(74) Representative: dompatent von Kreisler Selting Werner - Partnerschaft von Patent- und Rechtsanwälten mbB
(86) International application number: PCT/CN2014/083111
(87) International publication number: WO 2015/010659

(57) **Abstract**

Disclosed are an immuno-enhancer composition, a use of same as a vaccine adjuvant, and a preparation method therefor. The composition comprises rapamycin and a Toll-like receptor agonist-type adjuvant.

SEQUENCE LISTING

<110>  INSTITUT PASTEUR OF SHANGHAI, CHINESE ACADEMY OF SCIENCES

<120>  USE OF RAPAMYCIN AS VACCINE ADJUVANT AND PREPARATION METHOD THEREFOR

<130>  P2016-0004

<160>  24

<170>  PatentIn version 3.3

<210>  1
<211>  19
<212>  DNA
<213>  primer

<400>  1
   cacccttgcc ctcctaaac                                                    19

<210>  2
<211>  18
<212>  DNA
<213>  primer

<400>  2
cacctggcag gtccagag                                                      18

<210>  3
<211>  20
<212>  DNA
<213>  primer

<400>  3
agaccctgcc cattgaactg                                                    20

<210>  4
<211>  26
<212>  DNA
<213>  primer

<400>  4
gaagctggtg ctgtagttct catatt                                             26

<210>  5
<211>  23
<212>  DNA
<213>  primer

<400>  5
atttgaattc cctgggtgag aag                                                23

<210>  6
<211>  22
<212>  DNA
<213>  primer

<400>  6

cacaggggag aaatcgatga ca                                                22

<210>  7
<211>  23
<212>  DNA
<213>  primer

<400>  7
caccagcggg acatatgaat cta                                               23

<210>  8
<211>  22
<212>  DNA
<213>  primer

<400>  8
cagaactggc tgttgtcctt ga                                                22

<210>  9
<211>  25
<212>  DNA
<213>  primer

<400>  9
caaccaacaa gtgatattct ccatg                                             25

<210>  10
<211>  21
<212>  DNA
<213>  primer

<400>  10
gatccacact ctccagctgc a                                                 21

<210>  11
<211>  22
<212>  DNA
<213>  primer

<400>  11
gcctgcagat agttcccaaa ac                                                22

<210>  12
<211>  22
<212>  DNA
<213>  primer

<400>  12
gccatactcc accacgtaca ag                                                22

<210>  13
<211>  22
<212>  DNA
<213>  primer

<400>  13

ccattttcgg ggtgtttctc ta                                                22

<210>  14
<211>  22
<212>  DNA
<213>  primer

<400>  14
tgaccgggat aatcttccat ct                                                22

<210>  15
<211>  22
<212>  DNA
<213>  primer

<400>  15
aggattcggc gcagtaataa ca                                                22

<210>  16
<211>  22
<212>  DNA
<213>  primer

<400>  16
ccgaaggtaa ggctgttgtt tg                                                22

<210>  17
<211>  22
<212>  DNA
<213>  primer

<400>  17
tgtgttctgg aaacggagtc aa                                                22

<210>  18
<211>  22
<212>  DNA
<213>  primer

<400>  18
gaggagggcc acagtaactg aa                                                22

<210>  19
<211>  22
<212>  DNA
<213>  primer

<400>  19
gtccccaaag ggatgagaag tt                                                22

<210>  20
<211>  22
<212>  DNA
<213>  primer

<400>  20

gtttgctacg acgtgggcta ca                                              22

<210>  21
<211>  22
<212>  DNA
<213>  primer

<400>  21
cggtcaaaaa gtttgccttg ac                                              22

<210>  22
<211>  22
<212>  DNA
<213>  primer

<400>  22
gctcctcctt tccaggtcag tt                                              22

<210>  23
<211>  22
<212>  DNA
<213>  primer

<400>  23
tggagaaacc tgccaagtat ga                                              22

<210>  24
<211>  22
<212>  DNA
<213>  primer

<400>  24
ctgttgaagt cgcaggagac aa                                              22

## Description

### FIELD OF THE INVENTION

The present invention belongs to the field of immunology and pharmacy; in particular, the present invention relates to the use of rapamycin as a vaccine adjuvant and the preparation method thereof.

### BACKGROUND OF THE INVENTION

Application of a adjuvant is an important means to optimize the effect of an antigen. Adjuvants can not only quantitatively enhance the immune response induced by a vaccine, increase the titer of neutralizing antibody, but also qualitatively optimize the immune response, for example, improving the quantity of memory B cells and T cells and prolonging their survival time, so as to achieve superior protective effects compared with the immune response induced by "natural infection" itself. So far there are only three kinds of adjuvants for human, which are aluminium adjuvant, MF59 and mono-phosphate lipid A (MPL), and they can not meet the needs for so many vaccine researches. Therefore, the research and development for adjuvants are imperative. Aluminum adjuvant mainly enhances Th2-type antibody response, which is effective in clearing extracellular pathogens and parasitic infections; however, it is of little effect for treating RSV infection. MPL is only valid in the vaccine strategy against human papilloma virus HPV and hepatitis B virus HBV. Studies have shown that the stimulation of dendritic cells by MPL can only induce limited amount of IL-12, which makes it of limited capacity in enhancing Th1 response. The T cell-mediated immune responses and the secretion of IFNY have been proved to play a key role in clearing HIV, HCV and TB infection, and treating cancer. Therefore, the research and development for vaccine adjuvant strategy which can successfully induce T cell response is particularly urgent.

mTOR signaling pathway is a key pathway in the regulation of cell growth and proliferation which integrate signals from molecular nutrition, energy state and growth factor together so as to regulate a large number of life processes. The disorder of the pathway relates to a variety of human diseases, including cancer, diabetes and cardiovascular disease. Recent studies have shown that mTOR is also involved in the regulation of innate immunity and adaptive immunity, thus becoming a multi-functional regulating molecule. Rapamycin is a small molecular compound capable of specifically inhibiting mTOR, which has been approved by the United States FAD in 1999 for the anti-rejection therapy of renal transplantation, and it is also being studied for the use in treating cancer and other diseases.

### SUMMARY OF THE INVENTION

The object of the present invention is to provide a use of rapamycin as a vaccine adjuvant, and the preparation method thereof.

In the first aspect of the present invention, an immune enhancer composition is provided, comprising rapamycin or derivatives thereof, and Toll-like receptor (TLR) agonist adjuvant.

In a preferred embodiment, the Toll-like receptor agonist adjuvant comprises: Toll-like receptor 3 (TLR3), Toll-like receptor 4 (TLR4), Toll-like receptor 7 (TLR7), or combinations thereof.

In another preferred embodiment, the Toll-like receptor agonist adjuvant comprises: Monophosphoryl lipid A, lipopolysaccharide, poly I:C, R848, or combinations thereof.

In another preferred embodiment, in the immune enhancer composition, the weight ratio between rapamycin or derivatives thereof and Toll-like receptor agonist adjuvant is (0.2-20): 1; preferably (0.5-15): 1; more preferably (2-12): 1.

In another preferred embodiment, the immune enhancer composition further comprises: a pharmaceutically acceptable carrier (such as PBS or saline).

In another preferred embodiment, the amount of rapamycin or derivatives thereof and Toll-like receptor agonist adjuvant in the composition is an effective amount, e.g., 0.01-80% (W/W) of the composition; preferably 0.1-20% (w/w).

In another preferred embodiment, the immune enhancer composition further comprises antigens; preferably, the antigens include (but not limited to) inactivated or attenuated virus particles, virus like particles (VLP) or polypeptide antigens, protein antigens.

In another preferred embodiment, the weight ratio between the antigens and Toll-like receptor agonist adjuvant is (0.2-20): 1; preferably (0.5-10): 1; for example 1: 1, 2:1, 1:2.

In another preferred embodiment, the amount of antigens in the composition is an effective amount, e.g., 0.01-80% (w/w) of the composition; preferably 0.1-20% (w/w).

In another preferred embodiment, the antigens include (but not limited to): surface antigen of hepatitis B virus, antigen of hepatitis C virus, HIV antigens, tumor antigens or ovalbumins.

In another preferred embodiment, the derivative of rapamycin comprises (but not limited to): temsirolimus, everolimus, Deforolimus (AP23573), Zotarolimus , Ridaforolimus, CCI-779, RAD001, ABT-578, SDZ RAD, SAR 943, 1139-52 Biolimus A9 or other types of mTOR inhibitors which comprise (but not limited to) Ku0063794, PP242, PI-103, AZD8055, Palomid 529, AZD2014, Torin1, SNS-032.

In another aspect of the present invention, a kit is provided, which comprises the immune enhancer composition; or the kit comprises separately packaged rapamycin or derivatives thereof and Toll-like receptor agonist adjuvant.

In another preferred embodiment, the kit further comprises antigens.

In another aspect of the present invention, the use of rapamycin or derivatives thereof in the preparation of formulations, in which the activity of Toll-like receptor agonist adjuvant is enhanced, is provided.

In another aspect of the present invention, a method for producing the immune enhancer composition is provided, which comprises: mixing rapamycin or derivatives thereof and a Toll-like receptor agonist adjuvant so as to obtain the immunity enhancer composition.

In a preferred embodiment, rapamycin or derivatives thereof and Toll-like receptor agonist adjuvant are mixed at weight ratio of (0.2-20): 1; preferably (0.5-15): 1; more preferably (2-12): 1.

Other aspects of the present invention are obvious for those skilled in the art based on the disclosure herein.

### DESCRIPTION OF DRAWINGS

Figure 1, rapamycin enhanced the LPS-induced IL-12 and IL-23 production in BMDC, and inhibited the IL-10 production.
Figure 2, the influences of rapamycin on the gene expression induced by LPS in BMDM.
   (a) The influences of rapamycin on the transcriptional expression of Cytokine induced by LPS in BMDM;
   (b) The influences of rapamycin on the transcriptional expression of costimulatory molecule induced by LPS in BMDM;
   (c) The influences of rapamycin on the transcriptional expression of proinflammatory cytokines and chemokines induced by LPS in BMDM;

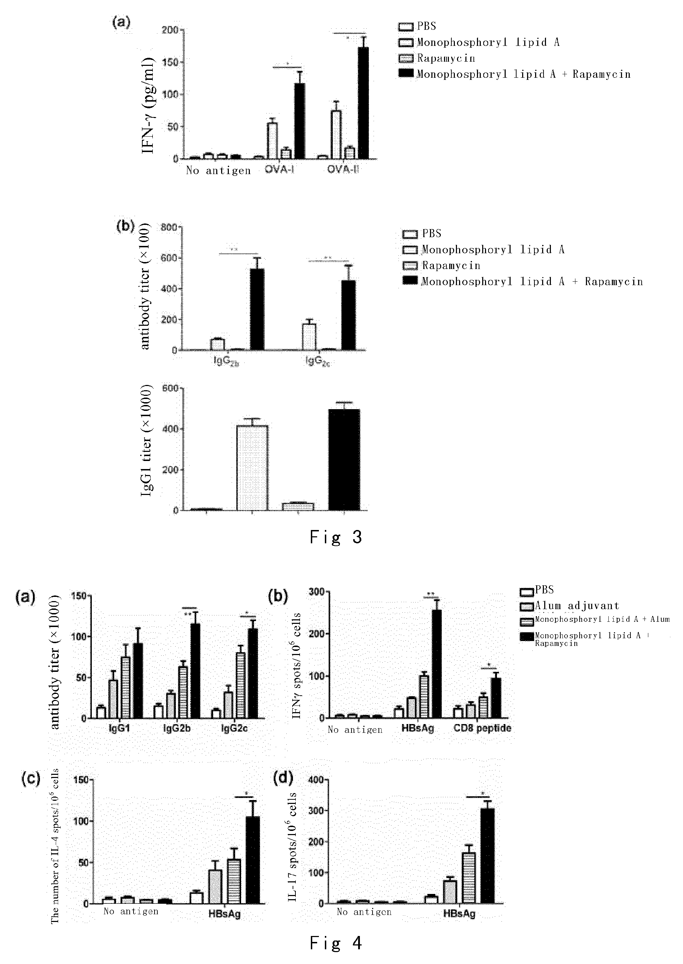

Figure 3, rapamycin enhanced the OVA-specific T cell responses and antibody responses.
   (a) rapamycin enhanced the OVA-specific Th1 and CD8+ cells responses;
   (b) rapamycin enhanced the OVA-specific IgG2b and IgG2c antibody titer;
Figure 4, rapamycin remarkably increased the adjuvant activity of MPL.
   (a) rapamycin increased the HBsAg specific antigen response enhanced by MPL;
   (b) rapamycin increased the HBsAg specific CD4+ and CD8+ T cell response enhanced by MPL;
   (c) rapamycin increased the HBsAg specific Th2 cell response enhanced by MPL;
   (d) rapamycin increased the HBsAg specific Th17 cell response enhanced by MPL.

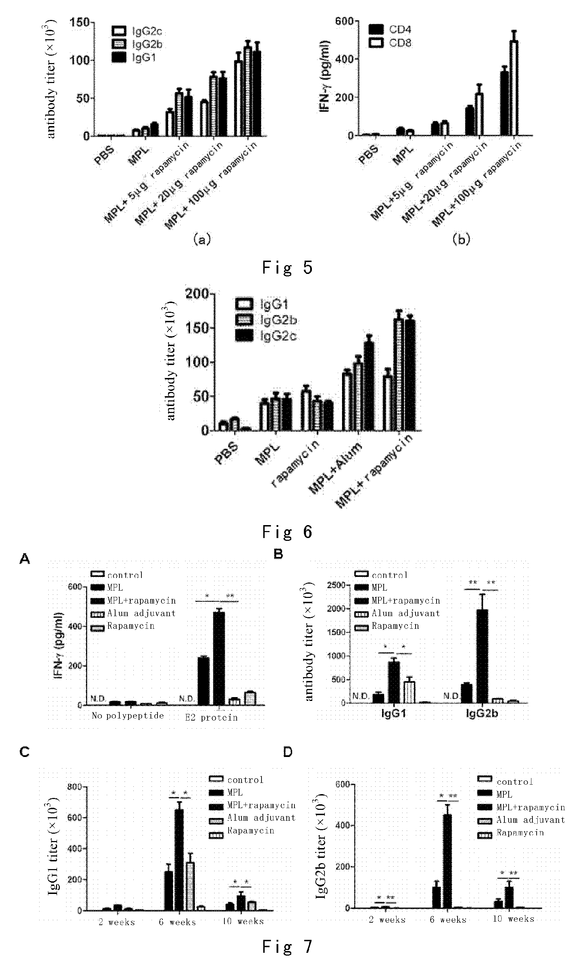

Figure 5, different amounts of rapamycin can remarkably increase the adjuvant activity of MPL.
   (a) Different amounts of rapamycin increased the HBsAg specific antigen response enhanced by MPL;
   (b) Different amounts of rapamycin increased the HBsAg specific CD4+ and CD8+ T cell response enhanced by MPL.
Figure 6, rapamycin can remarkably enhance the adjuvant activity of MPL in homology prime - boost immunization program.
Figure 7, rapamycin and MPL synergistically induced strong and persistent HCV immune response.
   A, rapamycin and MPL synergistically induced high level of T cell immune response against HCV E2.
   B, rapamycin and MPL synergistically induced high level of antibody response against HCV E2.
   C and D, rapamycin and MPL synergistically induced persistent antibody response against HCV E2.

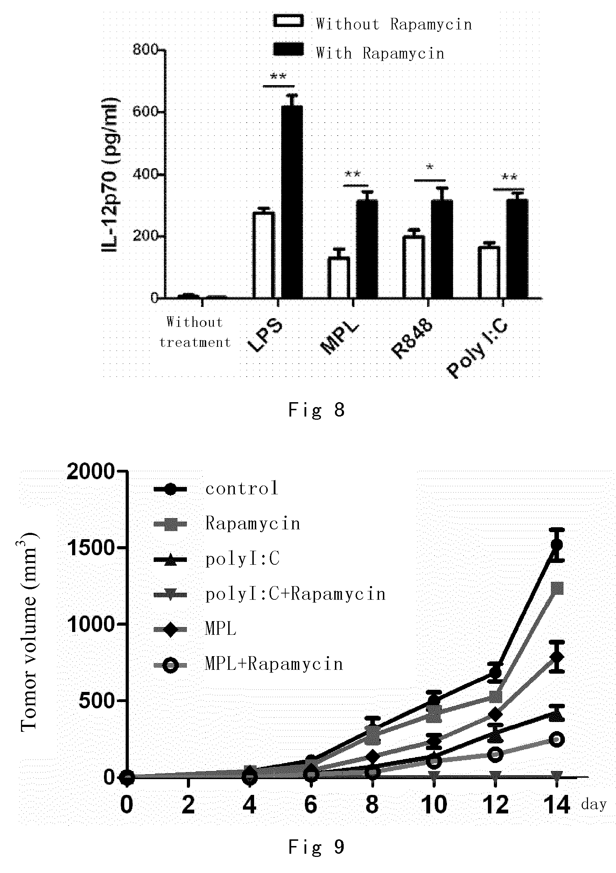

Figure 8, rapamycin effectively enhanced the IL-12 expression induced by MPL, polyI:C and R848.
Figure 9, rapamycin and MPL, polyI:C synergistically induced efficient immune response against tumor.

### EMBODIMENTS FOR CARRYING OUT THE INVENTION

Upon long and extensive research, the present inventors have surprisingly found that rapamycin possesses adjuvant activity which can promote the generation of some immune factors. Moreover, rapamycin will show synergistic effects when applied together with Toll-like receptor agonist adjuvant, such as a monophosphoryl lipid A (MPL), thereby greatly enhancing the immune response against the antigen in the body. The present invention is completed based on the above findings.

### Rapamycin and Uses Thereof

The molecular formula of rapamycin is C₅₁H₇₉NO₁₃, and the structure formula thereof is formula (I):

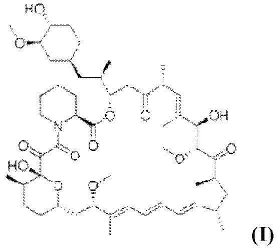

Derivatives of rapamycin can also be included in the present invention. The derivatives of the rapamycin include but not limited to: Temsirolimus, everolimus, Deforolimus (AP23573), Zotarolimus, Ridaforolimus , CCI-779, RAD001, ABT-578 , SDZ RAD, SAR 943, 1139-52 Biolimus A9, and other types of mTOR inhibitors, including but not limited to: Ku0063794, PP242, PI-103, AZD8055, Palomid 529, AZD2014, Torin1, SNS-032. These derivatives have similar structures to rapamycin (e.g., having similar nucleus structure) or similar mechanism, thus can be used in the present invention. For example, Koichi Araki 1 et al. (Nature. 2009 July 2; 460(7251): 108-1 12. doi: 10.1038/nature08155.mTOR regulates memory CD8 T cell differentiation) and Amiel E et al. (J Immunol. 2012 Sep 1; 189(5):2151-8. doi: 10.4049/jimmunol.1103741. Epub 2012 Jul 23. Inhibition of mechanistic target of rapamycin promotes dendritic cell activation and enhances therapeutic autologous vaccination in mice) have shown that mTOR inhibitors have similar mechanism.

Structures and formulas of some derivatives of rapamycin are listed hereinafter:
The molecular formula of Temsirolimus is C₅₆H₈₇NO₁₆, and the structure formula thereof is formula (II): 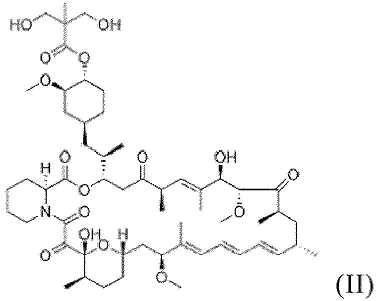
The molecular formula of Temsirolimus is C₅₆H₈₇NO₁₆, and the structure formula thereof is formula (III): 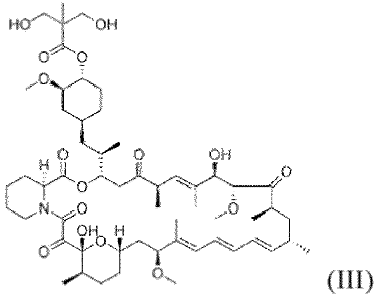
The molecular formula of everolimus is C₅₃H₈₃NO₁₄, and the structure formula thereof is formula (IV): 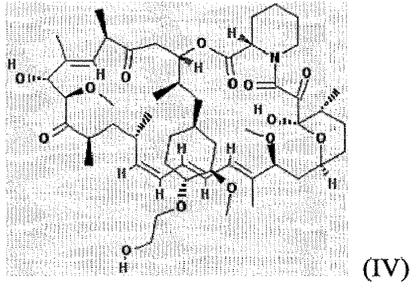
The molecular formula of 42-(dimethyl sulfoxide phosphonomethyl) rapamycin is C₅₃H₈₄NO₁₄P, and the structure formula thereof is formula (V): 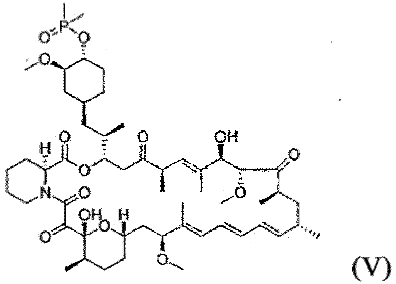
The molecular formula of 42-(dimethyl sulfoxide phosphonomethyl) rapamycin is C₅₃H₈₄NO₁₄P, and the structure formula thereof is formula (VI): 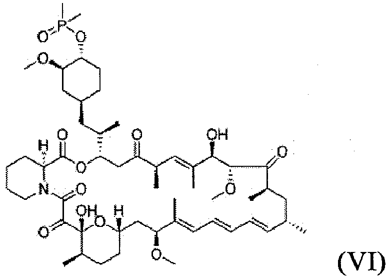
The molecular formula of Zotarolimus is C₅₂H₇₉N₅O₁₂, and the structure formula thereof is formula (VII): 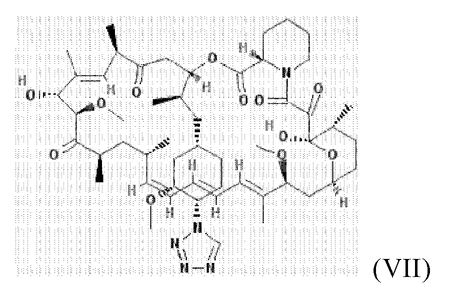
The molecular formula of Olcorolimus is C₅₁H₈₁NO₁₂, and the structure formula thereof is formula (VIII): 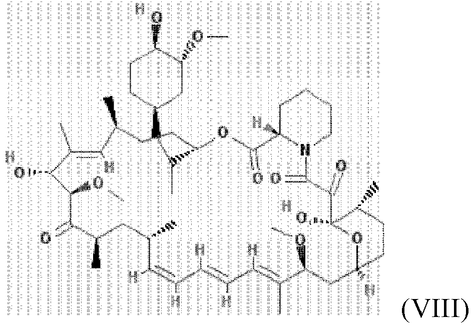
The molecular formula of Umirolimus is C₅₅H₈₇NO₁₄, and the structure formula thereof is formula (VIIII): 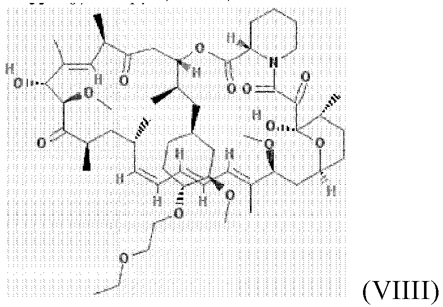
The molecular formula of Ku0063794 is C₂₅H₃₁N₅O₄, and the structure formula thereof is formula (X): 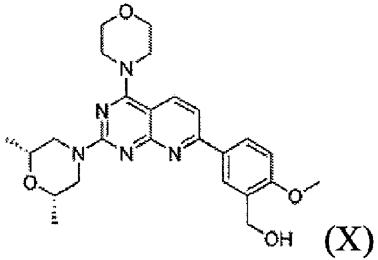
The molecular formula of PP242 is C₁₆H₁₆N₆O, and the structure formula thereof is formula (XI): 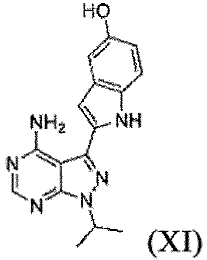
The molecular formula of PI-103 is C₁₉H₁₆N₄O₃, and the structure formula thereof is formula (XII): 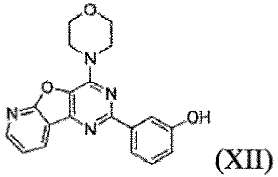
The molecular formula of AZD8055 is C₂₅H₃₁N₅O₄, and the structure formula thereof is formula (XIII): 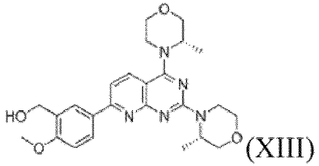
The molecular formula of Palomid 529 is C₂₄H₂₂O₆, and the structure formula thereof is formula (XIIII): 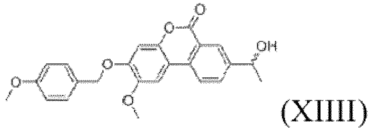
The molecular formula of AZD2014 is C₂₅H₃₀N₆O₃, and the structure formula thereof is formula (XV): 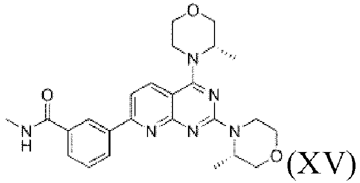
The molecular formula of Torin1 is C₃₅H₂₈F₃N₅O₂, and the structure formula thereof is formula (XVI): 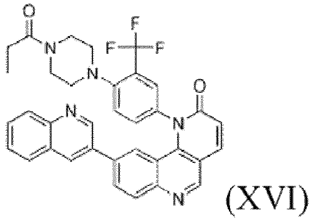
The molecular formula of SNS-032 is C₁₇H₂₄N₄O₂S₂, and the structure formula thereof is formula (XVII):

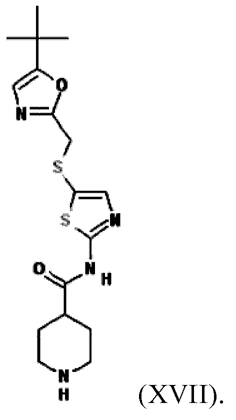

Currently, in the prior art, rapamycin or derivatives thereof are used as immunosuppressive drugs. They exert their effects by inhibiting G0 and G1 phase of cell cycle and blocking G1 phase entering into S phase through combining with the corresponding immunophilin RMBP, and their effects are listed as follows: ① inhibiting the proliferation of T and B cells; ② inhibiting lymphocyte proliferation induced by IL-1, IL-2, IL 6 and IFN-γ; ③ inhibiting the production of IgG and donor-specific antibody (cytotoxic antibodies); ④ inhibiting the proliferation of monocyte. They can be used in the anti-transplant rejection, and in the treatment of rheumatoid arthritis, lupus and other autoimmune diseases.

In the present invention, it was found that rapamycin or a derivative thereof can be used as an immune adjuvant. It has exhibited strong feasibility in terms of safety since it has been widely used in patients. The present invention has also demonstrated that rapamycin or derivatives thereof can significantly optimize the activity of Toll-like receptor agonist adjuvant; and in particular, it can enhance MPL induced T cell response.

Rapamycin or derivatives thereof are a type of commercial products, which is commercially available to the skilled person in the art. In addition, it can also be obtained through chemical synthesis.

Uses of rapamycin or derivatives thereof, or a pharmaceutically acceptable salt thereof are provided in the present invention for preparing immune enhancer; preferably, for preparing formulations for enhancing of activities of Toll-like receptor agonist adjuvants.

In order to confirm the above uses of rapamycin, the inventors, through *in vitro* cell tests, verified that rapamycin can promote dendritic cells to produce IL-12 and IL-23, inhibit the production of IL-10; promote the transcript expression of *IL-12a, IL-12b* and *IL-23α*; and inhibit the expression of *IL-10* and *PD*-*L1*.Furthermore, animal tests have confirmed that rapamycin can enhance the activity of a Toll-like receptor agonist adjuvant.

Therefore, rapamycin or derivatives thereof are a type of adjuvant which can effectively enhance antigen-specific T-cell and antibody response, and a combination of rapamycin or derivatives thereof and Toll-like receptor agonist adjuvant is even better than MPL and Aluminum Adjuvant which are the best in the present stage.

### Composition

Based on the novel discovery of the present invention, an immune enhancer composition comprising rapamycin or derivatives thereof and a Toll-like receptor agonist adjuvant is provided. The amounts of rapamycin or derivatives thereof and Toll-like receptor agonist adjuvant in the composition are both at safe and effective amount. As a preferred embodiment of the present invention, in the composition, the weight ratio between rapamycin or derivatives thereof and Toll-like receptor agonist adjuvant is (0.2-20): 1; preferably (0.5-15): 1; more preferably (2-12): 1. The composition can further comprise pharmaceutically acceptable carriers or excipients.

In the present invention, the "immune enhancer" means non-specific immune enhancer, which is also known as "adjuvant". When injected together with an antigen, or pre-injected into a body, the adjuvant can enhance the immune response against an antigen or change the type of immune response in the body.

In the present invention, the term "comprising" or "including" means that the various components can be used together in the mixture or composition of the present invention. The terms "mainly consisting of..." and "consisting of..." are included in the scope of the term "comprise".

In the present invention, the term "pharmaceutically acceptable" component is a substance which is suitable for applying to humans and / or animals without undue undesired side reactions (such as toxicity, stimulation or allergy), that is, a substance of reasonable benefit / risk ratio.

In the present invention, the term "pharmaceutically acceptable carrier" refers to a solvent, suspending agent or excipient acceptable in pharmaceutical or food which is used to deliver rapamycin or a derivative, or a physiologically acceptable salt thereof of the present invention to an animal or human. The carrier can be liquid or solid.

Dosage forms of the pharmaceutical composition according to the present invention can be varied, so long as the active ingredient can effectively get to the mammal. For example, it can be selected from: solutions, suspensions, powders, granules, tablets, capsules, syrups or aerosols, wherein rapamycin or derivatives thereof and / or Toll-like receptor agonist adjuvant may be present in a suitable solid or liquid carrier or dilution.

In the present invention, rapamycin or derivatives thereof and / or Toll-like receptor agonist adjuvant, and the compositions thereof may also be stored in a disinfected device suitable for injection or infusion.

The dose and mode for administering rapamycin or derivatives thereof and / or a Toll-like receptor agonist adjuvant may be varied. Toll-like receptor agonist adjuvant can be administered at 0.03-150 mg / 60 kg of body weight, preferably 3-60 mg / 60 kg of body weight; and rapamycin or derivatives thereof can be administered at 0.03-900 mg / 60 kg of body weight; preferably 0.3-600 mg / 60 kg of body weight. Generally, satisfying immune response can be induced by one treatment of the compounds of the present invention and the antigen, and even better effects can be achieved by repeating immunization for one time after a month. Booster immunization can be repeated for one time every 1-3 months, if necessary, or repeated 2-3 times so as to achieve the best immunity.

Rapamycin or derivatives thereof and / or Toll-like receptor agonist adjuvant may be comprised in a vaccine composition. The vaccine composition further comprises antigens to form a complete vaccine composition capable of inducing the immune response in the body.

The present invention will be further illustrated below with reference to the specific examples. It should be understood that these examples are only to illustrate the invention but not to limit the scope of the invention. The experimental methods with no specific conditions described in the following examples are generally performed under the conventional conditions, for example, according to J. Sambrook et al., Molecular Cloning: A Laboratory Manual, 3rd Edition, Science Press, 2002, or according to the manufacture's instructions. Unless indicated otherwise, parts and percentage are calculated by weight.

### Example 1. Rapamycin Remarkably Enhancing the Induced-production of Il-12 And Il-23 in Dendritic Cells

Cytokines IL-12, which is secreted by dendritic cells, is a major regulatory factor for inducing Th1 and CD8. MPL, as a ligand for TLR4, merely stimulate dendritic cells to induce limited IL-12.The inventors firstly tested by *in vitro* experiment whether rapamycin can enhance IL-12 production induced by activation of TLR4 signaling in dendritic cells.

Bone marrow stem cells from 6-8 weeks aged C57BL/6 mice were taken, and erythrocytes were removed by using ammonium chloride lysate (0.15 M NH₄Cl, 1 mM KHCIO₃, 0.1 mM Na₂EDTA, pH7.3), and then suspension-cultured at a cell density of 10⁶ cells / mL in a dendritic cell (bone-marrow derived dendritic cell, BMDC) differentiation medium ( formulation of which was: RPMI 1640 complete medium (comprising 5%(v/v) FBS and 1%(v/v) pen/strep), with 20 ng/ml GM-CSF, 10 ng/ml 1L-4, 2 mM L-glutamine and 200 µM β-mercaptoethanol being added). After differentiation for 7 days, the cells were purified with CD 11c magnetic beads to obtain BMDC. The purified BMDC were resuspended in a fresh dendritic cell culture medium (10⁶ cells/ml), and inoculated into 24-well plate with 500 µL per well. 100 nM of rapamycin or DMSO (control) were used to pre-treat the cells for 1 hour, and then the cells were stimulated with 100 ng/ml LPS for 24 hours. The supernatant was taken and the amount of IL-12 was measured by ELISA.

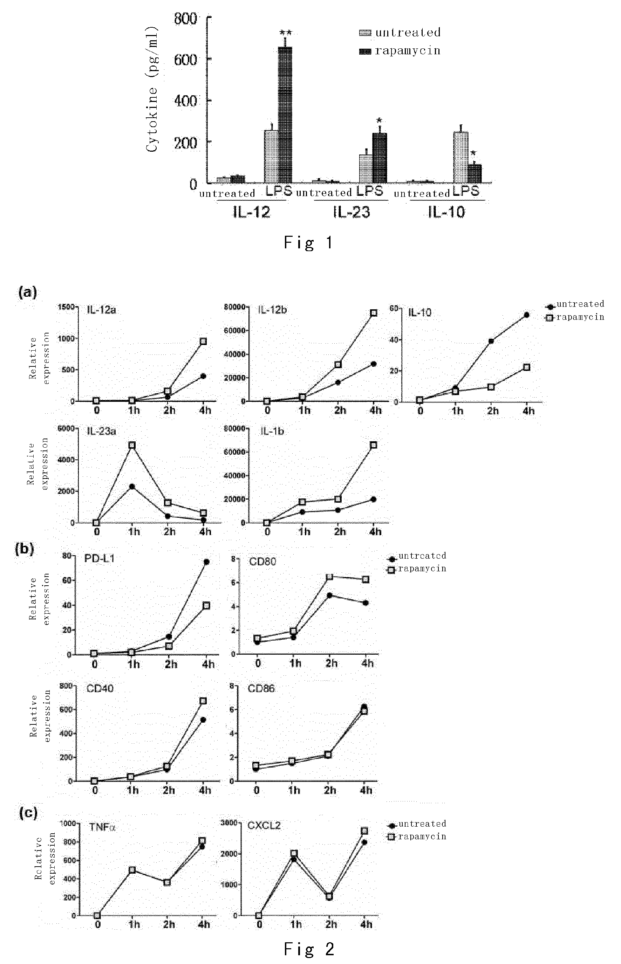

The results were shown in fig 1, that is, after pre-treated with rapamycin, the amount of IL- 12 was increased by more than 3 times compared with the control group. Moreover, the inventors have further found that rapamycin can remarkably increase the induced-secretion of IL-23 of BMDC, and in contrast, inhibit the production of IL-10.

### Example 2. Rapamycin selectively affected relevant genes regulating adaptive immunity without affecting the expression of pro-inflammatory cytokines

Bone marrow stem cells of 6-8 weeks aged C57BL/6 mice were taken, and the cells are cultivated at 10⁶ cells / mL cell density in bone marrow-derived macrophage (BMDM) differentiation medium (formulation of which was: RPMI 1640 complete medium added with 30% (v/v) L929 conditioned medium and 1%(v/v) pen/strep). After differentiation for 4 days, the old culture medium (comprising suspended cells) was removed and fresh BMDM differentiation medium was added. The adhered BMDMs were collected on the 7^{th} day of differentiation and used in *in vitro* experiment.

The BMDMs were pre-treated with 100 nM of rapamycin or DMSO for 1 hour, and then the cells were stimulated with 100 ng/ml LPS for 24 hours. The cells were collected at 1, 2, 4 and 8 hours, and were cryopreserved in a refrigerator at -80°C for the extraction and preparation of RNA.

TRIZOL (Invitrogen) method was used to extract RNA of BMDM, and Superscript™ III Reverse Transcriptase Kit (Invitrogen) was used to respectively reverse transcribe 1 µg RNA so as to synthesize the first-strand cDNA for Real-time quantitative PCR determine of gene expression levels. The primers for Real-time quantitative PCR are shown in table 1. ΔΔ method was used to calculate the relative expression level of the determined gene compared with GAPDH.

**Table 1. Primers for Real-time quantitative PCR**

| Gene | forward primer (5'-3') | SEQ ID NO: | reverse primer (5'-3') | SEQ ID NO: |
|---|---|---|---|---|
| *IL-12a* | CACCCTTGCCCTCCTAAAC | 1 | CACCTGGCAGGTCCAGAG | 2 |
| *IL-12b* | AGACCCTGCCCATTGAACTG | 3 | GAAGCTGGTGCTGTAGTTCTCA TATT | 4 |
| *IL-10* | ATTTGAATTCCCTGGGTGAGAA G | 5 | CACAGGGAGAAATCGATGAC A | 6 |
| *IL-23a* | CACCAGCGGGACATATGAATCT A | 7 | CAGAACTGGCTGTTGTCCTTGA | 8 |
| *IL*-*1β* | CAACCAACAAGTGATATTCTCC ATG | 9 | GATCCACACTCTCCAGCTGCA | 10 |
| *PD-L1* | GCCTGCAGATAGTTCCCAAAAC | 11 | GCCATACTCCACCACGTACAAG | 12 |
| *CD40* | CCATTTTCGGGGTGTTTCTCTA | 13 | TGACCGGGATAATCTTCCATCT | 14 |
| *CD80* | AGGATTCGGCGCAGTAATAAC A | 15 | CCGAAGGTAAGGCTGTTGTTTG | 16 |
| *CD86* | TGTGTTCTGGAAACGGAGTCAA | 17 | GAGGAGGGCCACAGTAACTGA A | 18 |
| *TNFα* | GTCCCCAAAGGATGAGAAGT T | 19 | GTTTGCTACGACGTGGGCTACA | 20 |
| *CXCL2* | CGGTCAAAAAGTTTGCCTTGAC | 21 | GCTCCTCCTTTCCAGGTCAGTT | 22 |
| *GAPDH* | TGGAGAAACCTGCCAAGTATG A | 23 | CTGTTGAAGTCGCAGGAGACA A | 24 |

The results of real-time quantification PCR were shown in figure 2, that is, during 4 hours of LPS stimulation, rapamycin increased the transcriptional expression of IL-12a, IL-12b and lL-22a, but inhibit the expression of IL-10 (figure 2a).

Furthermore, the inventors have also found that some costimulatory molecules and cytokines involved in adaptive immune response were also affected by rapamycin, and exhibited different expression patterns within the time frame of the process. Figure 2 has shown that, upon stimulation of LPS, rapamycin remarkably increased the expression of PD-L1, while has no remarkable influence on CD80, CD86 and CD40.

Moreover, rapamycin did not affect the transcription and expression of pro-inflammatory cytokines or chemokines such as TNFα and CXCL2 (Figure 2c), which indicated that rapamycin is of low possibility to cause non-specific inflammation when used as an adjuvant.

### Example 3. Rapamycin Remarkably Enhanced the Adjuvant Activity of MPL

Rapamycin could significantly enhance the production of IL-12(p70) induced by LPS both in dendritic cells and macrophages, but how about it's ability to regulate antigen-specific adaptive immune response? 6-8 weeks aged mice were primarily immunized by the inventors using intraperitoneal injection method, wherein Ovalbumin (10µg/mouse) was used as antigen and PBS (negative control), MPL (10 µg/mouse, positive control), rapamycin (100 µg/mouse), rapamycin (100 µg/mouse) +MPL (10 µg/mouse) as adjuvant respectively, and were immunized again with ovalbumin (10 µg/mouse, without adjuvant) at 3 weeks. The mice were killed 1 week after the secondary immunization, and blood was taken to determine the content of OVA specificity IgG1, IgG2b, IgG2c; meanwhile, 10 µg/ml of OVA-1 (SIINFEKL) or OVA-II (ISQAVHAAHAEINEAGR, Invitrogen) was used to stimulate splenic cells (5×10⁵ cells). After 48 hours, enzyme linked immunosorbent assay (ELISA) was used to determine the content of IFNγ in the supernatant.

The results have shown that (figure 3) MPL, as an adjuvant, can significantly increase OVA-specific CD4 + and CD8 + T cell responses secreting IFNγ and IgG2b, IgG2c and IgG1 antibody titers. Comparing with the negative control, rapamycin alone can produce comparatively moderate OVA-specific CD4 + and CD8 + T cell responses and IgG2b, IgG2c and IgG1 antibody titers. What is important is that when compared with MPL alone, the combination of rapamycin and MPL has exhibited nearly 2 times of increase in OVA-specific CD4 + and CD8 + T cell responses, while the OVA-specific IgG2b, IgG2c antibody titers were also significantly increased. OVA-specific IgG1 antibody titer did not shown obvious difference under MPL or MPL+rapamycin used as adjuvant. These results have shown that rapamycin can effectively enhance the adjuvant effect of MPL.

Virus-like particles (VLP) consisting of (HBV) outer membrane protein antigens is one of the few successful recombinant vaccines clinically used. Aluminium adjuvant (Alum) can only enhance the immune response of VLP in the elderly people and patients with renal dysfunction, while it is of limited effects. However, Alum together with MPL can significantly increase the activity of HBV vaccine. The previous results of the inventors have shown that rapamycin can effectively enhance the adjuvant effect of MPL. In order to further analyze the adjuvant activity of rapamycin, C57BL/6 mice were immunized by the inventors using HBsAg VLP (5 µg /mouse) (Shanghai Yemin Biotechnology Co., Ltd., Item number YM-101) as antigen in mixture with Alum (200 µg /mouse), Alum (200 µg /mouse)+MPL (10 µg /mouse), MPL(10 µg /mouse)+rapamycin (100 µg /mouse) respectively, and was immunized with HBsAg (without MPL or rapamycin) 3 weeks after the primary immunization. The mice were killed 2 weeks after the secondary immunization, and blood was taken to determine the content of HBsAg-specific IgG1, IgG2b, IgG2c. To test HBsAg-specific T cell responses, the inventors have determined HBsAg-specific induced Th1, Th17 and Th2 response by ELISpot (Elispot Assay).

The specific operations are listed as follows: spleen cells were inoculated at 5×10⁵ per-well into Elispot 96-wells plate of IFNγ, IL-17 or IL-4, and treated or untreated (control) with HBsAg (10 µg/ml) or CD8 peptide fragment (10 µg/ml, ILSPFLPLL, VWLSVIWM, China Peptides Co., LTD., Shanghai), incubated under 37°C, 5% CO₂ for two days. The cells and supernatants were removed, The plate was washed with PBST for 5 times, then incubated with 50 µl of 2 µg/ml biotin-coupled IFNγ (ebioscience, cat No: 13-7312) IL-17 (ebioscience, cat No: 13-7177) or IL-4 (Mabtech, cat No:3311-6-250) detecting antibody under 37°C for 2 hours, washed with PBST for 5 times, then incubated with 100 µl of 1000-fold diluted Streptavidin-alkaline phosphatase (Mabtech, cat No:3310-10) under 37°C for 1 hour, washed with PBST for 3 times, and then washed with PBS for another two times. 100 µl of NBT/CPIT substrate (Mabtech, cat No: 3650-10) was added and incubated at room temperature for 0.5 hour, and then washed with deionized water for 3 times, and was naturally dried in darkness. The number of spot was read with ELISPOT READER, and was statistically analyzed.

The results (figure 4) have shown that when compared with MPL+Alum immunized group, rapamycin+MLP can significantly increase the HBsAg-specific IgG2b, IgG2c antibody titers (figure 4a) and CD4 +, CD8 + T cell responses (figure 4b). Moreover, the inventors have found that MPL+rapamycin immunized group has shown stronger Th2 (figure 4c) and Th17 (figure 4d) cell response compared with MPL+Alum immunized group.

Based on the above results, rapamycin is a type of adjuvant which could effectively enhance the antigen-specific T cell and antibody responses, while the combination of rapamycin and MPL is even better than the MPL and aluminum adjuvant, which is the best at this stage.

### Example 4. Composition of Rapamycin and MPL

Composition 1, comprising (dosage for one mouse): rapamycin 5 µg, MPL 10 µg, and PBS 0.2ml;
Composition 2, comprising (dosage for one mouse): rapamycin 20 µg, MPL 10 µg, and PBS 0.2ml;
Composition 3, comprising (dosage for one mouse): rapamycin 100 µg, MPL 10 µg, and PBS 0.2mL.

Mice were immunized with Compositions 1-3 in mixture with HBsAg (5 µg, dosage for one mouse) respectively, and were boosted 3 weeks after the primary immunization. The mice were killed after one week, and the serum was prepared to determine the content of HBsAg-specific IgG1, IgG2b and IgG2c (figure 5). Meanwhile, spleen cells (5×10⁵ cells) were respectively stimulated by using 10 µg/ml of CD8⁺T cell epitope peptide (ILSPFLPLL or VWLSVIWM) and CD4+ T cell epitope peptide (RGLYFPAGGSSSG), and after 48 hours, enzyme linked immunosorbent assays (ELISA) was used to determine the content of IFNγ in the supernatant (figure 5b). The results have shown that 5 µg of rapamycin can significantly increase the antibody titer induced by MPL, which increased the secretion of IgG2b and IgG2c by nearly 5 times. When the dosage of rapamycin was raised to 20 or 100 µg, the antigen expression induced by MPL can be further enhanced (figure 5a). Moreover, at all of the three concentrations, rapamycin has increased T cell response induced by MPL, especially the activation of CD4+and CD8+T cells which secrete IFN-γ (figure 5b). Therefore, rapamycin has shown outstanding immune-enhancing effect within the range of 5-100 µg, therefore it can promote MPL-induced antibody titers and T cell response in a dose-dependent manner. This result suggests that rapamycin as an adjuvant can be used in a lager dose range, and its effect is proportional to dose within a certain range.

### Example 5. Use of Compositions of Rapamycin and MPL in Repeated Immunization can Significantly Enhance the Effect of a Vaccine

HBsAg (5 µg/mouse) was respectively mixed with the following adjuvants or adjuvant combinations: rapamycin (100 µg/mouse) + MPL (10 µg/mouse), MPL (10 µg/mouse), rapamycin (100 µg/mouse) or Alum (200 µg/mouse) + MPL(10 µg/mouse) to immunize (primary immunization) C57BL6 mice. After 4 weeks, each group of mice was boosted according to the same program as the primary immunization. The mice were killed after two weeks, and serum was prepared for determining the content of HBsAg-specific IgG1, IgG2b and IgG2c. Figure 6 has shown that rapamycin, used as an adjuvant, can significantly enhance the HBsAg-induced antibody titers after immunized for two times, including the contents of IgG1, IgG2b and IgG2c. Compared with MPL alone, combinations of rapamycin, Alum and MPL provided better effects, indicating that the combination of the two adjuvants has excellent synergistic effects. Especially, the combination of rapamycin and MPL is even better than the combination of MPL and Alum.

Similarly, when derivatives of rapamycin were used to replace rapamycin for combining with MPL as above, it was found that the rapamycin derivatives can also significantly enhance the HBsAg-induced antibody titers, which provided significant effects as compared with MPL alone.

### Example 6. Combination of Rapamycin and MPL can Enhance the Effects of HCV Vaccine

Hepatitis C virus HCV infects about 3-4 million people per year, and there is no effective preventive or therapeutic vaccines at present. Since IFN-γ secreting T cells and broad-spectrum neutralizing antibodies are considered as necessary immune response in preventing the HCV infection, and the previous data have shown that rapamycin can enhance MPL-induced T cell immune response and antibody titers, therefore, the present inventors have further studied whether the combination of rapamycin and MPL can induce an high strength immune response against HCV outer membrane protein E2.

C57BL/6 mice were immunized using purified E2 protein (20 µg/mouse, brought from eEnzyme company, Cat No. HCV-E21b) as antigen in mixture with Alum(200 µg/mouse), MPL(10 µg/mouse), rapamycin (100 µg/mouse), MPL(10 µg/mouse) + rapamycin(100 µg/mouse) respectively, and after 4 weeks, were boosted by the same antigen+adjuvant program. 8 weeks after the secondary immunization, mice were immunized for one time with E2 protein (without MPL or rapamycin) (5 µg/mouse), and the mice were killed after 2 weeks. Spleen cells were taken to determine immune response of T cells (figure 7A), and serum was taken to determine antibody titers (figure 7B). The spleen cells (5×10⁵ cells) were stimulated with 10 µg/ml of E2 protein, and after 48 hours, the content of IFNγ in the supernatant was determined by enzyme linked immunosorbent assay (ELISA). It was found that the combination of rapamycin and MPL induced the highest immune response of IFN-γ secreting T cells, followed by MPL, while alum or rapamycin alone provided inferior effect (figure 7A). Meanwhile, antibody response induced by the combination of rapamycin and MPL was also the best, which was better than MPL or alum alone (Figure 7B). More importantly, the serum was taken at different time points after primary immunization to determine the contents of E2 specific IgG1 and IgG2b (figure 7C-D), and it was found that the combination of MPL and rapamycin not only can quickly induce antibody response (better antibody response can be achieved after two weeks from the primary immunization); but also the antibody response induced by them can last for a longer time of period, in which strong antibody titers can be detected in the tenth week after immunization (figure 7C and D).

These results indicate that rapamycin and MPL as adjuvant was capable of inducing strong and sustained T cell and antibody responses, which is the best candidate adjuvant for new HCV vaccine.

### Example 7. Combination of Rapamycin and MPL or polyI:C can Enhance Antitumor Immunity

polyI:C, as an immune adjuvant, can effectively induce cytotoxic T cell immune response, therefore, it has been widely used in cancer vaccines. But tumor vaccines with polyI:C as adjuvant has not yet been clinically used. One possible reason is that the strength and durability of induced Cytotoxic T cell immune response needed to be improved.

The present inventors have firstly studied the influence of rapamycin on IL-12 expression induced by a variety of Toll-like receptors agonist including polyI: C by *in vitro* experiments. Dendritic cells differentiated from mouse bone marrow cells (BMDC differentiated for 7 days with 20 ng/ml GM-CSF and 10 ng/ml IL-4) were pre-treated with or without rapamycin (100nM) for 1 hour, and stimulated by LPS (100 ng/ml), MPL (1 µg/ml), R848 (1 µg/ml) and polyI:C (20 µg/ml) respectively for 24 hours, and cell culture fluid was taken to determine the content of IL-12 by ELISA. The results have shown that not only can rapamycin enhance the IL-12 expression induced by lipopolysaccharide and MPL (TLR4 ligand), but also can it effectively enhance polyI: C (TLR3 ligand) and R848 (TLR7 ligand) induced IL-12 expression, as shown in Figure 8.

The inventors have further explored the ability of rapamycin to enhance MPL and polyI:C-induced Cytotoxic T-cell immune responses and the anti-tumor effect. By intraperitoneal injecting, 6-8 weeks aged mice were immunized using ovalbumin (10 µg/mouse) as antigen and PBS (200 µl, negative control), polyI:C (Invivogen) (50 µg/mouse), rapamycin (100 µg/mouse), MPL (10 µg/mouse), MPL(10 µg/mouse) + rapamycin (100 µg/mouse), polyI:C (50 µg/mouse) + rapamycin (100 µg/mouse) used as adjuvant to conduct primary immunization. After two weeks, the mice were boosted by the same program. 4 weeks after the secondary immunization, melanoma murine cell line B16 OVA expressing OVA was subcutaneous vaccinated into mice (1×10⁶ cells/mouse), and the tumor growth was measured. In the mice of control group without adjuvant being added, the tumor normally grew and the tumor volume increased with time (Figure 9). In contrast, in the mice immunized with polyI:C as an adjuvant, the tumor grew slowly and was of relatively small volume. While in the mice immunized with polyI:C + rapamycin as adjuvant, the tumor growth was completely inhibited (figure 9). Experimental results have shown that the adjuvant combinations of rapamycin and polyI:C has an excellent result in enhancing anti-tumor immune response. It also shows that the combination of rapamycin and MPL also has significant anti-tumor effect (Figure 9).

All literatures mentioned in the present application are incorporated herein by reference, as though each one is individually incorporated by reference. Additionally, it should be understood that after reading the above teachings, those skilled in the art can make various changes and modifications to the present invention. These equivalents also fall within the scope defined by the appended claims.

## Claims

1. An immune enhancer composition, wherein the composition comprises rapamycin or derivatives thereof, and a Toll-like receptor agonist adjuvant.

2. The immune enhancer composition according to claim 1, wherein the Toll-like receptor agonist adjuvant comprises: Toll-like receptor 3, Toll-like receptor 4, Toll-like receptor 7, or combinations thereof.

3. The immune enhancer composition according to claim 2, wherein the Toll-like receptor agonist adjuvant comprises: Monophosphoryl lipid A, lipopolysaccharide, polyI:C, R848, or combinations thereof.

4. The immune enhancer composition according to claim 1, wherein the weight ratio between rapamycin or derivatives thereof and Toll-like receptor agonist adjuvant is (0.2-20): 1; preferably (0.5-15): 1; more preferably (2-12): 1.

5. The immune enhancer composition according to claim 1, wherein the immune enhancer composition further comprises a pharmaceutically acceptable carrier.

6. The immune enhancer composition according to claim 4, wherein the immune enhancer composition further comprises antigens; preferably, the antigens include: inactivated or attenuated virus particles, virus like particles (VLP) or polypeptide antigens, protein antigens.

7. The immune enhancer composition of claim 6, wherein the antigen comprising: surface antigen of hepatitis B virus, hepatitis C virus antigens, HIV antigens, tumor antigens or ovalbumin.

8. The immune enhancer composition of claim 1, wherein the derivatives of rapamycin comprise: temsirolimus, everolimus, Deforolimus, Zotarolimus , Ridaforolimus, CCI-779, RAD001, ABT-578 , SDZ RAD, SAR 943, 1139-52 Biolimus A9 or other types of mTOR inhibitors which comprise Ku0063794, PP242, PI-103, AZD8055, Palomid 529, AZD2014, Torin1, or SNS-032.

9. A kit, wherein the kit comprises the immune enhancer composition of any one of claims 1-8; or
the kit comprises separately packaged rapamycin or derivatives thereof and Toll-like receptor agonist adjuvant.

10. The kit according to claim 9, wherein the Toll-like receptor agonist adjuvant comprises: Toll-like receptor 3, Toll-like receptor 4, Toll-like receptor 7, or combinations thereof; preferably, the Toll-like receptor agonist adjuvant comprises: Monophosphoryl lipid A, lipopolysaccharide, poly I:C, R848, or combinations thereof.

11. The kit of claim 9, wherein the kit further comprises an antigen.

12. The kit of claim 9, wherein the derivatives of rapamycin comprise: temsirolimus, everolimus, Deforolimus, Zotarolimus , Ridaforolimus, CCI-779, RAD001, ABT-578 , SDZ RAD, SAR 943, 1139-52 Biolimus A9 or other types of mTOR inhibitors which comprise Ku0063794, PP242, PI-103, AZD8055, Palomid 529, AZD2014, Torin1, or SNS-032.

13. Use of rapamycin or a derivative thereof in the preparation of formulations in which the activity of Toll-like receptor agonist adjuvant is enhanced.

14. The use according to claim 13, wherein the Toll-like receptor agonist adjuvant comprises: Toll-like receptor 3, Toll-like receptor 4, Toll-like receptor 7, or combinations thereof; preferably, the Toll-like receptor agonist adjuvant comprises: Monophosphoryl lipid A, lipopolysaccharide, poly I:C, R848, or combinations thereof.

15. The use of claim 13, wherein the derivatives of rapamycin comprises: temsirolimus, everolimus, Deforolimus, Zotarolimus , Ridaforolimus, CCI-779, RAD001, ABT-578 , SDZ RAD, SAR 943, 1139-52 Biolimus A9 or other types of mTOR inhibitors which comprise Ku0063794, PP242, PI-103, AZD8055, Palomid 529, AZD2014, Torin1, or SNS-032.

16. A method for preparing the immune enhancer composition of claim 1,
wherein the method comprises: mixing the rapamycin or derivatives thereof and Toll-like receptor agonist adjuvant so as to obtain the immune enhancer composition.

17. The method according to claim 16, wherein rapamycin or derivatives thereof and Toll-like receptor agonist adjuvant is mixed at weight ratio of (0.2-20): 1; preferably (0.5-15): 1; more preferably (2-12): 1.

18. The method of claim 16, wherein the derivatives of rapamycin comprise: temsirolimus, everolimus, Deforolimus, Zotarolimus , Ridaforolimus, CCI-779, RAD001, ABT-578 , SDZ RAD, SAR 943, 1139-52 Biolimus A9 or other types of mTOR inhibitors which comprise Ku0063794, PP242, PI-103, AZD8055, Palomid 529, AZD2014, Torin1, or SNS-032.

19. The method according to claim 16, wherein the Toll-like receptor agonist adjuvant comprises: Toll-like receptor 3, Toll-like receptor 4, Toll-like receptor 7, or combinations thereof; preferably, the Toll-like receptor agonist adjuvant comprises: Monophosphoryl lipid A, lipopolysaccharide, poly I:C, R848, or combinations thereof.
